# EUROPEAN PATENT SPECIFICATION

(11) **EP 3 749 270 B1**
(45) Date of publication and mention of the grant of the patent: **13.03.2024**
(21) Application number: 18804431.7
(22) Date of filing: 05.02.2018
(51) Int. Cl.: A61F 13/44, A61B 90/00

(54) **A SURGICAL PAD AND A METHOD OF OBTAINING THE SAME**
CHIRURGISCHES KISSEN UND VERFAHREN ZU SEINER HERSTELLUNG
TAMPON CHIRURGICAL ET SON PROCÉDÉ D'OBTENTION

(43) Date of publication of application: 16.12.2020
(73) Proprietor: Yeditepe Üniversitesi, Istanbul (TR)
(72) Inventor: YASARGIL, Mahmut Gazi, Istanbul (TR); YASARGIL, Dianne Cathryn Helena, Istanbul (TR); ATALAY, Basar, Istanbul (TR)
(74) Representative: Dericioglu, E. Korhan
(86) International application number: PCT/TR2018/050040
(87) International publication number: WO 2019/151961

(56) References cited:
- WO-A1-97/40797
- US-A- 3 911 922
- US-A- 4 477 256
- US-A- 4 639 253
- US-A- 5 575 781
- US-A1- 2007 219 516

## Description

### FIELD OF THE INVENTION

The present invention relates to surgical pad bunches which are particularly used in microsurgical operations such as brain and spinal cord operations.

### STATE OF THE ART

As is known, in surgical operations, particularly in brain and spinal cord surgical operations, pads are used to absorb bodily fluids such as blood, lymph, and cerebrospinal fluid. In addition to the requirement that the pads used in these operations should be small in size, they should also reflect X-rays, i.e. should be radiopaque, in order to be detected radiologically during the surgery. Furthermore, there should not be any sticking on the parts of the pads contacting the tissue.

The state of the art patent application no. US3911922 discloses a surgical sponge which is made of a porous fabric coated on both sides with a flexible foamed polymer, both surfaces of which can absorb liquids. The sponge comprises an X-ray detectable material.

Another patent application no. EP2641560A discloses a surgical pad, which is coated with a resin that prevents any residues from the pad surface to remain on the site during an operation, is comprised of a plurality of absorbent fabric structure layered on top of one another, and has a layer containing X-ray radiopaque substance.

Another patent application no. US2005109347 discloses a surgical towel comprising an X-ray detectable material. The surgical towel is made of a sheet of woven fabric having an X-ray detectable material. The X-ray detectable material is sewn or otherwise attached to the fabric. The hem is stitched with a thread of a different color to show that the towel comprises an X-ray detectable material.

Further surgical pads with X-ray detectable material are disclosed in US 5575781 A, US4477256 A, US4639253 A, WO97/40797 A1 and US2007/219516 A1.

### SUMMARY OF THE INVENTION

The object of the present invention is to provide a surgical pad bunch with surgical pads, which are suitable in size for use in microsurgical operations such as brain and spinal cord surgical operations and which do not stick to the body tissue, and a surgical pad bunch obtaining method.

In order to achieve the said object, the invention is a surgical pad bunch formed by connecting tail parts of a plurality of surgical pads and where the surgical pad having a head part including an intermediate layer comprising an X-ray detectable material for use in surgical operations, particularly in brain and spinal cord operations. In the surgical pad, the intermediate layer is provided between layers, which are produced from 100% cotton and have a geometric shape that is manually formed. Thus, a radiopaque surgical pad, which is of desired small sizes for microsurgical operations, and which does not stick to the body tissue, is provided.

In a possible embodiment of the invention, the head part is produced from a pressed cotton or a hydrophilic cotton material. This way, a head part that does not stick to the body tissue is obtained.

In another possible embodiment of the invention, the head part has a square, rectangular or circular geometric shape. Thus, surgical pads of different geometric shapes are provided for microsurgical operations.

Another possible embodiment of the invention comprises a tail part connected to the head part. Thus, a tail part is provided for the surgical pads.

In another possible embodiment of the invention, the tail part is stitched to the head part such that it is marked with an X-ray detectable material. Thus, the surgical pad is marked with a radiopaque stitch.

In another possible embodiment of the invention, the tail part is a thread produced from a cotton material. This way, the tail part is produced from a material that does not stick to the body tissue.

According to the invention, a surgical pad bunch is formed by connecting the tail parts of a plurality of surgical pads. Thus, a surgical pad bunch comprising a plurality of surgical pads connected to each other is obtained, for packaging.

In a possible embodiment of the invention, the X-ray detectable material is an indicator comprising barium oxide or ion.

In order to achieve the said object, the invention is a method of obtaining surgical pad bunch from surgical pad having an angular geometry for use in surgical operations, particularly brain and spinal cord operations. The method of obtaining a surgical pad comprises the steps of manually folding a pressed cotton (milk filter) along the length thereof to form an upper layer and a lower layer and cutting them to predetermined lengths, placing an X-ray detectable material between the upper layer and the lower layer to form a radiopaque intermediate layer, cutting at least one edge part of the pressed cotton to acquire the final form and obtaining the head part, stitching a cotton thread to the head part such that it is marked with an X-ray detectable material and thus forming a tail part, as the final procedure, a surgical pad bunch is obtained by connecting a plurality of surgical pads through at least one connection point. Thus, a method of manually obtaining surgical pad bunches is introduced.

In an alternative embodiment of the invention, the lower and upper layers at the head part of the surgical pad can be a milk filter produced from cotton material. In another alternative embodiment of the invention, the surgical pad can be obtained in different geometric shapes which are angular or not.

According to the invention, the surgical pad bunch is obtained by connecting the tail parts of a plurality of surgical pads for packaging.

### BRIEF DESCRIPTION OF THE FIGURES

Figure 1 is a representative view of the surgical pad having a circular head part.
Figure 2 is a representative view of the surgical pad bunch comprising surgical pads having circular head parts for packaging.
Figure 3 is a representative view of the surgical pad having a rectangular head part.
Figure 4 is a representative view of the surgical pad bunch comprising surgical pads having rectangular head parts for packaging.
Figure 5 is a representative view showing the layers forming the head part of the surgical pad.

### DETAILED DESCRIPTION OF THE INVENTION

In this detailed description, a surgical pad (10) and a method for obtaining the said surgical pad (10) are described by examples that are only provided to facilitate better understanding of the invention without producing any limiting effect.

The surgical pad (10), which is used for absorbing the bodily fluids in microsurgical operations such as brain and spinal cord operations, is basically comprised of a head part (11) an a tail part (12). The head part (11) is produced from a material that will enable the surgical pads (10) to absorb bodily fluids. An end of the tail part (12) is connected to the head part (11) by stitching from a stitching part (13). The other end of the tail part (12) is free.

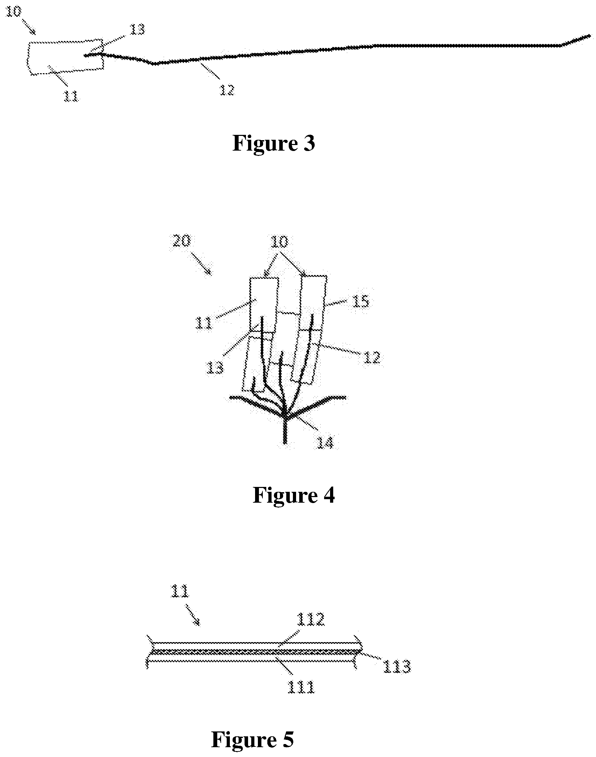

Referring to Figure 5, the head part (11) includes a lower layer (111), and an upper layer (112) corresponding to the lower layer (111). The upper layer (112) and the lower layer (111) are produced from 100% cotton and have a geometric shape that is manually formed. In more detail, the said %100 cotton material is pressed cotton or hydrophilic cotton. In a possible embodiment, the head part (11) may have a square, rectangular or circular shape. An intermediate layer (113) comprising an X-ray detectable material is provided between the said lower layer (111) and upper layer (112). In a possible embodiment, the X-ray detectable material is an indicator comprising barium oxide or ion.

The surgical pad (10) having a rectangular head part (11) given in Figure 3 is obtained by means of the following method. First of all, the pressed cotton is manually folded along the length thereof so as to produce the upper layer (112) and the lower layer (111). Then the pressed cotton is cut from the folded parts to predetermined lengths by the help of a cutter. Thus the lower layer (111) and the upper layer (112) are provided such that they have matching geometries. An intermediate layer (113) is formed between the said upper and lower layers (111, 112) by placing an X-ray detectable material. At least one edge part (15) of the pressed cotton is cut to acquire the final form and thus the head part (11) is obtained. A cotton thread is stitched to the head part (11) such that it is marked with an X-ray detectable material and this way, the tail part (12) and the head part (11) are connected to each other. Thus a rectangular surgical pad (10) is obtained. As the final procedure, referring to Figure 4, a plurality of surgical pads (10) are connected through at least one connection point (14) thereby providing a surgical pad bunch (20). The said surgical pad bunch (20) has a similar appearance with a grape bunch. The head parts (11) of the surgical pads (10) in the surgical pad bunch (20) at least partially contact each other and are positioned so as to neighbor each other. In a possible embodiment, the surgical pad bunch (20) is provided by connecting at least five surgical pads (10) to each other. The said head part (10) can have any angular geometrical shape such square and rectangle.

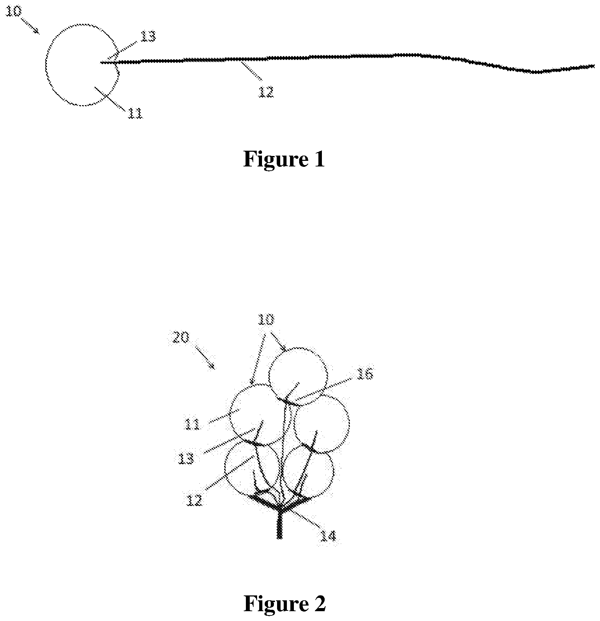

The surgical pad (10) having a circular head part (11) given in Figure 1 is obtained by means of the following method. First of all, the hydrophilic cotton is manually formed into a geometric shape at desired pad sizes. An X-ray detectable material is placed on the hydrophilic cotton to form an intermediate layer (113). The said hydrophilic cotton will form the lower layer (111) and the upper layer (112) of the head part (11). When the hydrophilic cotton is wrapped around the X-ray detectable material, the X-ray detectable material is positioned between the upper layer (112) and the lower layer (111). Then, the lower layer (111) and the upper layer (112) are connected to the each other when an end part (16) of the head part (11) which is open is stitched. This way, the head part (11) is formed into a disc having an upper layer (112) and lower layer (111). The said end part (16) is provided in at least one part of the periphery of the hydrophilic cotton. The head part (11) is obtained when the portions exceeding the periphery of the hydrophilic cotton are cut such that the hydrophilic cotton will acquire its final form. A cotton thread is stitched to the head part (11) such that it is marked with an X-ray detectable material and thus the tail part (12) and the head part (11) of the surgical pad (10) are connected to each other. Thus a circular surgical pad (10) is obtained. As the final procedure, referring to Figure 2, a plurality of surgical pads (10) are connected through at least one connection point (14) thereby providing a surgical pad bunch (20). The said surgical pad bunch (20) has a similar appearance with a grape bunch. The head parts (11) of the surgical pads (10) in the surgical pad bunch (20) at least partially contact each other and are positioned so as to neighbor each other. In a possible embodiment, the surgical pad bunch (20) is provided by connecting at least five surgical pads (10) to each other. The said head part (11) can have any geometric shape without corners such as a circle. The surgical pad (10) and the surgical pad bunch (20) obtained by means of any of the above mentioned methods are sterilized thereby obtaining surgical pads (10) that are suitable to be used in surgical operations.

### REFERENCE NUMBERS

10. Surgical pad
11. Head part
111. Lower layer
112. Upper layer
113. Intermediate layer
12. Tail part
13. Stitching part
14. Connection point
15. Edge part
16. End part
20. Surgical pad bunch

## Claims

1. A surgical pad bunch (20) formed by connecting tail parts (12) of a plurality of surgical pads (10) and where the surgical pad (10) having a head part (11) including an intermediate layer (113) comprising an X-ray detectable material for use in surgical operations, particularly in brain and spinal cord operations, the intermediate layer (113) is provided between layers (111, 112), which are produced from 100% cotton and have a geometric shape that is manually formed.

2. A surgical pad bunch (20) according to Claim 1 **characterized in that** the head part (10) is produced from a pressed cotton or a hydrophilic cotton material.

3. A surgical pad bunch (20) according to Claim 1 or 2 **characterized in that** the head part (11) has a square, rectangular or circular geometric shape.

4. A surgical pad bunch (20) according to any one of the preceding claims **characterized in that** the surgical pad comprises a tail part (12) connected to the head part (11).

5. A surgical pad bunch (20) according to Claim 4 or 5 **characterized in that** the tail part (12) is a thread produced from a cotton material.

6. A surgical pad bunch (20) according to any one of the preceding claims, **characterized in that** the X-ray detectable material is an indicator comprising barium oxide or ion.

7. A method of obtaining surgical pad bunch (20) from surgical pad (10) having an angular geometry for use in surgical operations, particularly brain and spinal cord operations, **characterized in that** it comprises the steps of
- manually folding a pressed cotton along the length thereof to form an upper layer (112) and a lower layer (111) and cutting them to predetermined lengths,
- placing an X-ray detectable material between the upper layer (112) and the lower layer (111) to form a radiopaque intermediate layer (113),
- cutting at least one edge part (15) of the pressed cotton to acquire the final form and obtaining the head part (11),
- stitching a cotton thread to the head part (11) such that it is marked with an X-ray detectable material and thus forming a tail part (12),
- as the final procedure, a surgical pad bunch (20) is obtained by connecting a plurality of surgical pads (10) through at least one connection point (14).

8. A method of obtaining surgical pad bunch (20) from surgical pad (10) having a non-angular geometry for use in surgical operations, particularly brain and spinal cord operations, **characterized in that** it comprises the steps of
- manually forming the hydrophilic cotton into a geometric shape at desired pad sizes,
- placing an X-ray detectable material into the hydrophilic cotton to form a radiopaque intermediate layer (113),
- wrapping the hydrophilic cotton around the X-ray detectable material, stitching an end part (16), and forming it into a disc having an upper layer (112) and a lower layer (111),
- obtaining the head part (11) having a non-angular geometry by cutting the portions exceeding the periphery of the hydrophilic cotton such that the hydrophilic cotton acquires its final form,
- as the final procedure, a surgical pad bunch (20) is obtained by connecting a plurality of surgical pads (10) through at least one connection point (14).
- stitching a cotton thread to the head part (11) such that it is marked with an X-ray detectable material and thus forming a tail part (12).

## Patentansprüche

1. Ein OP-Unterlagenbündel (20) zur Verwendung bei chirurgischen Operationen, insbesondere bei Gehirn- und Rückenmarksoperationen, das durch Verbinden von Schwanzteilen (12) einer Vielzahl von OP-Unterlagen (10) gebildet wird, wobei die OP-Unterlage (10) einen Kopfteil (11) mit einer Zwischenschicht (113) aufweist, die ein röntgendetektierbares Material umfasst, wobei die Zwischenschicht (113) zwischen Schichten (111, 112) vorgesehen ist, die zu 100 % aus Baumwolle hergestellt sind und eine geometrische Form haben, die manuell geformt ist.

2. Ein OP-Unterlagenbündel (20) nach Anspruch 1, **dadurch gekennzeichnet, dass** der Kopfteil (10) aus gepresster Baumwolle oder einem hydrophilen Baumwollmaterial hergestellt ist.

3. Ein OP-Unterlagenbündel (20) nach Anspruch 1 oder 2, **dadurch gekennzeichnet, dass** der Kopfteil (11) eine quadratische, rechteckige oder kreisförmige geometrische Form aufweist.

4. Ein OP-Unterlagenbündel (20) nach einem der vorangehenden Ansprüche, **dadurch gekennzeichnet, dass** die OP-Unterlage einen Schwanzteil (12) umfasst, der mit dem Kopfteil (11) verbunden ist.

5. Ein OP-Unterlagenbündel (20) nach Anspruch 4 oder 5, **dadurch gekennzeichnet, dass** der Schwanzteil (12) ein aus Baumwollmaterial hergestellter Faden ist.

6. Ein OP-Unterlagenbündel (20) nach einem der vorangehenden Ansprüche, **dadurch gekennzeichnet, dass** das röntgendetektierbare Material ein Indikator ist, der Bariumoxid oder -ionen umfasst.

7. Verfahren zur Herstellung eines OP-Unterlagenbündels (20) aus OP-Unterlagen (10) mit einer eckigen Geometrie zur Verwendung bei chirurgischen Eingriffen, insbesondere bei Gehirn- und Rückenmarkoperationen, **dadurch gekennzeichnet, dass** es die folgenden Schritte umfasst
- manuelles Falten eines gepressten Baumwollstoffs entlang dessen Länge, um eine obere Schicht (112) und eine untere Schicht (111) zu bilden, und anschließendes Zuschneiden auf vorbestimmte Längen,
- Anbringen eines röntgendetektierbaren Materials zwischen der oberen Schicht (112) und der unteren Schicht (111), um eine röntgenfähige Zwischenschicht (113) zu bilden,
- Abschneiden mindestens eines Randabschnitts (15) der gepressten Baumwolle, um die endgültige Form zu erhalten und den Kopfteil (11) zu erzeugen,
- Aufnähen eines Baumwollfadens auf den Kopfteil (11), sodass dieser mit einem röntgendetektierbaren Material markiert ist und somit einen Schwanzteil (12) bildet,
- als abschließender Schritt wird ein OP-Unterlagenbündel (20) erhalten indem eine Vielzahl von OP-Unterlagen (10) über mindestens einen Verbindungspunkt (14) miteinander verbindet werden.

8. Verfahren zur Herstellung eines OP-Unterlagenbündels (20) aus OP-Unterlagen (10) mit einer nicht-eckigen Geometrie zur Verwendung bei chirurgischen Operationen, insbesondere bei Gehirn- und Rückenmarksoperationen, **dadurch gekennzeichnet, dass** es die folgenden Schritte umfasst
- manuelles Formen der hydrophilen Baumwolle in eine geometrische Form mit gewünschten Unterlagengrößen,
- Anbringen eines röntgendetektierbaren Materials in die hydrophile Baumwolle, um eine röntgenfähige Zwischenschicht (113) zu bilden,
- Wickeln der hydrophilen Baumwolle um das röntgendetektierbare Material, Aufnähen eines Endteils (16) und Formen desselben in eine Scheibe mit einer oberen Schicht (112) und einer unteren Schicht (111),
- Herstellen des Kopfteils (11) mit einer nicht-eckigen Geometrie durch Abschneiden derjenigen Abschnitte, die über den Umfang der hydrophilen Baumwolle hinausgehen, sodass die hydrophile Baumwolle ihre endgültige Form erhält,
- als abschließender Schritt wird ein OP-Unterlagenbündel (20) erhalten indem eine Vielzahl von OP-Unterlagen (10) über mindestens einen Verbindungspunkt (14) miteinander verbindet werden,
- Aufnähen eines Baumwollfadens auf den Kopfteil (11), sodass dieser mit einem röntgendetektierbaren Material markiert ist und somit einen Schwanzteil (12) bildet.

## Revendications

1. Un tas de tampons chirurgicaux (20) formé en connectant les parties arrière (12) d'une pluralité de tampons chirurgicaux (10) et où le tampon chirurgical (10) ayant une partie tête (11) comprend une couche intermédiaire (113) comprenant un matériau détectable aux rayons X pour une utilisation dans les opérations chirurgicales, en particulier dans les opérations du cerveau et de la moelle épinière, la couche intermédiaire (113) est fournie entre les couches (111, 112), qui sont produites à partir de 100% de coton et ont une forme géométrique qui est formée manuellement.

2. Un tas de tampons chirurgicaux (20) selon la revendication 1, **caractérisé en ce que** la partie tête (10) est fabriquée à partir d'un coton pressé ou d'un matériau de coton hydrophile.

3. Un tas de tampons chirurgicaux (20) selon la revendication 1 ou 2, **caractérisé en ce que** la partie tête (11) a une forme carrée, rectangulaire ou géométrique.

4. Un tas de tampons chirurgicaux (20) selon une quelconque de revendications précédentes, **caractérisé en ce que** le tampon chirurgical comprend une partie arrière (12) connectée à la partie tête (11).

5. Un tas de tampons chirurgicaux (20) selon la revendication 4 ou 5, **caractérisée en ce que** la partie de l'arrière (12) est un fil produit à partir d'un matériau en coton.

6. Un tas de tampons chirurgicaux (20) selon une quelconque de revendications précédentes, **caractérisé en ce que** le matériau détectable aux rayons X est un indicateur comprenant de l'oxyde de baryum ou l'ion.

7. Une méthode de l'obtention d'un tas de tampons chirurgicaux (20) à partir d'un tampon chirurgical (10) ayant une géométrie angulaire pour une utilisation dans des opérations chirurgicales, en particulier des opérations sur le cerveau et la moelle épinière, **caractérisée en ce qu'**elle comprend les étapes suivantes :
- plier manuellement un coton pressé sur sa longueur pour former une couche supérieure (112) et une couche inférieure (111) et les couper à des longueurs prédéterminées,
- placer un matériau détectable aux rayons X entre la couche supérieure (112) et la couche inférieure (111) pour former une couche intermédiaire radio-opaque (113),
- couper au moins une partie du bord (15) du coton pressé pour obtenir la forme finale et obtenir la partie de tête (11),
- coudre un fil de coton à la partie de tête (11) de manière à ce qu'elle soit marquée d'un matériau détectable aux rayons X et former ainsi une partie d'arrière (12),
- comme procédure finale, un tas de tampons chirurgicaux (20) est obtenue en connectant plusieurs tas de tampons chirurgicaux (10) par au moins un point de connexion (14).

8. Une méthode d'obtention d'un tas de tampons chirurgicaux (20) à partir des tampons chirurgicaux (10) ayant une géométrie non angulaire pour une utilisation dans des opérations chirurgicales, en particulier des opérations sur le cerveau et la moelle épinière, **caractérisée en ce qu'**elle comprend les étapes suivantes :
- former manuellement le coton hydrophile en une forme géométrique aux dimensions souhaitées du tampon,
- placer un matériau détectable aux rayons X dans le coton hydrophile pour former une couche intermédiaire radio-opaque (113),
- envelopper le coton hydrophile autour du matériau détectable aux rayons X, coudre une partie terminale (16), et former un disque ayant une couche supérieure (112) et une couche inférieure (111),
- obtenir la partie de tête (11) ayant une géométrie non angulaire en coupant les parties dépassant la périphérie du coton hydrophile de manière à ce que le coton hydrophile acquière sa forme finale,
- comme procédure finale, un tas de tampons chirurgicaux (20) est obtenu en connectant plusieurs tampons chirurgicaux (10) à travers au moins un point de connexion (14),
- coudre un fil de coton à la partie de tête (11) de manière à ce qu'elle soit marquée d'un matériau détectable aux rayons X et former ainsi une partie d'arrière (12).
